## Europäisches Patentamt
## European Patent Office
## Office européen des brevets

(19)

(11) Veröffentlichungsnummer: **0 233 970**
**B1**

(12)

# EUROPÄISCHE PATENTSCHRIFT

(45) Veröffentlichungstag der Patentschrift: 20.06.90

(51) Int. Cl.⁵: **G01N 33/46**

(21) Anmeldenummer: **86102332.3**

(22) Anmeldetag: **22.02.86**

(54) **Schnittholz-Prüfvorrichtung.**

(43) Veröffentlichungstag der Anmeldung:
**02.09.87 Patentblatt 87/36**

(45) Bekanntmachung des Hinweises auf die Patenterteilung:
**20.06.90 Patentblatt 90/25**

(84) Benannte Vertragsstaaten:
**AT BE CH DE FR GB IT LI NL SE**

(56) Entgegenhaltungen:
EP-A- 0 160 781
WO-A-85/00657
DE-U- 8 135 891
GB-A- 2 115 141
US-A- 3 976 384

ANALYTICAL CHEMISTRY, Band 56, Nr. 4, April 1984,
Seiten 742-745, New York, US; W.E. NEELEY
"Reflectance photometer for multilayer dry film slides"

(73) Patentinhaber: **Helmut K. Pinsch GmbH & Co.,
Theodorstrasse 41h, D-2000 Hamburg 50(DE)**

(72) Erfinder: **Hatje, Günter H., Wellingsbüttler Weg 182,
D-2000 Hamburg 65(DE)**

(74) Vertreter: **Richter, Joachim, Dipl.-Ing. et al,
Patentanwälte Richter, Werdermann & Gerbaulet Neuer
Wall 10, D-2000 Hamburg 36(DE)**

## Beschreibung

Die Erfindung betrifft eine Schnittholz-Prüfvorrichtung für die laufende Erfassung von gesägtem Schnittholz, mit mindestens einer Lichtquelle, die das Schnittholz in einer Ebene quer zu seiner Längsrichtung mit im wesentlichen gleicher Intensität beleuchtet, mit mindestens einer optischen Erfassungsvorrichtung, die ansprechend auf Helligkeits- und/oder Farbänderungen in der von der Erfassungsvorrichtung erfaßten beleuchteten Ebene des Schnittholzes während einer Relativbewegung zwischen Schnittholz und optischer Erfassungsvorrichtung ein Informationssignal abgibt, und mit einer mit der Erfassungsvorrichtung verbundenen Informationssignale zu jedem geprüften Stück Schnittholz sequentiell abspeicherbar sind, wobei eine Anpaßschaltung vorgesehen ist, mit der eine Fehlerschwelle für die Erzeugung des Informationssignals einstellbar ist.

Bei bekannten Schnittholz-Prüfvorrichtungen wird einseitig ein mechanischer Impuls an das zu prüfende Schnittholz-Brett abgegeben. Mittels mehrerer Sensoren, die in vorbestimmten Abständen an dem Brett angebracht sind, wird der Amplitudenverlauf der dadurch erzeugten Stoßwelle gemessen. Die von den Sensoren abgegebenen Daten werden in einen Prozeßrechner eingegeben, der aus den gemessenen Durchlaufzeiten den E-Modul zwischen den Sensoren ermittelt. Aus Abweichungen von den Standardwerten kann auf das Vorliegen eines Holzfehlers in dem Brett geschlossen werden.

Dieses Verfahren läßt sich jedoch nur bei Brettern anwenden, die die gleiche Länge haben. Soll die Produktion für die nächste Charge auf andere Längen umgestellt werden, ist jeweils eine relativ aufwendige Anpassung erforderlich.

Zudem können mit diesem bekannten Verfahren nicht alle auftretenden Holzfehler in gleicher Weise zuverlässig erfaßt werden. Hierzu gehören Farbfehler, die nicht erfaßt werden können. Ein weiterer Nachteil besteht darin, daß die Sensoren an jedem zu messenden Brett erneut angebracht werden müssen. Ein kontinuierlicher Betrieb ist mit dem oben genannten Verfahren nicht möglich.

Es ist auch bereits ein optisches Erfassungsgerät der eingangs genannten Art zum Erfassen von Schädigungen in gesägtem oder behobeltem Holz bekannt.

Dieses betrifft dabei besonders die Erfassung von Schädigungen wie Astknoten, Verblauungen und bestimmte Arten von Fäule, um das automatische Klassifizieren von Holz zu erleichtern. Hierzu wird eine Prüfvorrichtung vorgeschlagen, bei der das in einer Längsrichtung bewegte Schnittholz von einer Lichtquelle beleuchtet wird, die das Schnittholz in einer Ebene quer zu seiner Längsrichtung mit im wesentlichen gleicher Intensität beleuchtet, wobei eine optische Erfassungsvorrichtung vorgesehen ist, die ansprechend auf Helligkeits- und/oder Farbänderungen in der von der Erfassungsvorrichtung erfaßten beleuchteten Ebene des Schnittholzes während einer Realtivbewegung zwischen Schnittholz und optischer Erfassungseinrichtung ein Informationssignal abgibt. Dabei ist eine mit der Erfassungsvorrichtung verbundene Rechenvorrichtung vorgesehen, mit der positionsbezogene Informationssignale zu jedem geprüften Stück Schnittholz sequentiell abspeicherbar sind, wobei eine Anpaßschaltung vorgesehen ist, mit der eine Fehlerschwelle für die Erzeugung des Informationssignales einstellbar ist (US-A 3 976 384).

Eine derart arbeitende Schnittholz-Prüfvorrichtung ist mit verschiedenen Nachteilen verbunden, die dazu führen, daß die Durchlaufgeschwindigkeit des gesägten Schnittholzes nicht erhöht bzw. nicht auf die Geschwindigkeit gebracht werden kann, die erforderlich wäre, um beispielsweise in Sägewerken oder holzverarbeitenden Betrieben anfallenden Hölzer und Schnitthölzer zu qualifizieren. Hierbei ist zu berücksichtigen, daß die Sägeleistung der Hochleistungsband- bzw. Gattersäge derart gestiegen ist, daß eine ungeheuer große Oberfläche pro Zeiteinheit zu qualifizieren ist. Um auf der anderen Seite dem wachsenden Kostendruck im Hinblick auf verwendete Rohstoffe entgegentreten zu können, ist es auch wünschenswert, Partien von grundsätzlich minderwertigem Schnittholz so zu qualifizieren, daß die höherwertigen Abschnitte ausgesondert und einer entsprechenden Verwendung zugeführt werden können. Auch hierbei ist der Wunsch gegeben, möglichst schnell und optimal eine Qualifizierung der einzelnen Abschnitte eines Schnittholzes vornehmen zu können, damit einerseits aufgrund der Qualifizierung die Einleitung in die entsprechende Verarbeitungslinie erfolgen kann und andererseits anhand der aufgenommenen Daten zur Lage von Fehlstellen das Trennen der optimalen Abschnitte von den weniger guten Abschnitten innerhalb der nachfolgenden Verarbeitungslinie erfolgen kann.

Hierzu ist es allerdings notwendig, daß die gesamte Oberfläche eines Schnittholzes, d.h. sowohl die Gesamtheit der Oberfläche an einer Seite als auch die Oberfläche aller Seiten möglichst schnell, vollständig und genau überprüft werden können, damit den voranstehenden Anforderungen nachgekommen werden kann.

Bei technischen Prüfvorrichtungen ist es bereits vorgesehen worden, Erfassungsvorrichtung(en) und Lichtquelle(n) an einen geeigneten Tragrahmen anzuordnen. So ist es bereits für eine Vorrichtung zur Ermittlung und Auswertung von Schwankungen des Flächengewichts von Vliesen vorgeschlagen worden, eine obere und eine untere Erfassungsvorrichtung mit einer Lichtquelle an einem Tragrahmen anzuordnen (DE-U 8 135 891).

Bei anderen bekannten Schnittholz-Prüfvorrichtungen ist eine Rechenvorrichtung vorgesehen, die dazu geeignet ist, aus dem geprüften Stück Schnittholz den fehlerfreien Teil auszuwählen (WO 85/00657).

Bei einem weiteren Oberflächenuntersuchungsgerät ist bereits vorgesehen worden, ein Umlenkspiegelsystem mit nur einem optischen Gerät anstelle einer Vielzahl von gleichen optischen Geräten vorzusehen (GB-A 2 115 141).

Bei einer anderen bekannten Einrichtung zum Prüfen von Leiterbahnen auf Leiterbahnplatten ist vorgesehen, die Leiterbahnplatte in einer Ebene quer zur Leiterlängsrichtung zu beleuchten, wobei ein schmaler Bereich beleuchtet wird, der mittels einer als CCD-Meßzeile ausgebildeten optischen Erfassungsvorrichtung zeilenweise abgetastet wird (EP-A 160 781).

Die bekannten Vorrichtungen sind jedoch für die Holzprüfung jeweils mit verschiedenen Nachteilen behaftet, da sie keinen entsprechend gezielten Aufbau aufweisen und insbesondere nicht die in diesem Bereich wünschenswerten hohen Durchlaufgeschwindigkeiten ermöglichen. Beispielsweise innerhalb eines Sägewerkes ist es jedoch so, daß die Sägeeinrichtungen derart große Mengenleistungen ermöglichen, daß die Durchlaufmenge im wesentlichen von der Geschwindigkeit der Qualifizierung abhängt.

Die Aufgabe der vorliegenden Erfindung besteht daher darin, eine Prüfvorrichtung für Schnittholz zu schaffen, bei der auftretende Holzfehler, und zwar unabhängig von ihrer Lage auf der Holzoberfläche, zuverlässig, und zwar unabhängig von der Stärke und Größe des Fehlers und schnell, d.h. ohne eine Abbremsung der Bewegung des Holzes in Durchlaufrichtung erfaßt werden können, so daß ein kontinuierlicher Betrieb möglich ist und darüber hinaus ein Erfassen von Fehlstellen, stück- und lagebezogen möglich ist, so daß geeignete Abschnitte von gewünschter fehlerfreier Länge schnell erfaßt werden können, um Schnittholz geringer Güteklasse für höherwertige Zwecke verwenden zu können.

Zur Lösung dieser Augabe wird eine Schnittholz-Prüfvorrichtung der eingangs genannten Art vorgeschlagen, die erfindungsgemäß in der Weise ausgebildet ist, daß

a) die Schnittholz-Prüfvorrichtung einen Tragrahmen umfaßt, an dem eine obere optische Erfassungseinrichtung und zwei seitliche optische Erfassungsvorrichtungen mit je einer Lichtquelle und eine untere optische Erfassungsvorrichtung mit einer weiteren Lichtquelle angeordnet sind, oder einen Tragrahmen umfaßt, der ein Lichtquelle, eine optische Erfassungsvorrichtung und eine Anzahl von Umlenkspiegeln trägt, die derart an dem Tragrahmen angeordnet sind, daß die Lichtquelle das Schnittholz mit im wesentlichen gleicher Intensität beleuchtet, wobei die optische Erfassungsvorrichtung über das Umlenkspiegelsystem die beleuchteten Ebenen des Schnittholzes erfaßt,

b) die jeweilige Lichtquelle das Schnittholz in der Ebene quer zu seiner Längsrichtung kegelförmig beleuchtet, wobei ein schmaler und sich über die jeweilige Breite des Schnittholzes erstreckender Bereich beleuchtet wird,

c) in dieser Ebene die mittels einer CCD-Meßzeile zeilenweise abtastende optische Erfassungsvorrichtung vorgesehen ist,

d) die Rechenvorrichtung eine Zeit- oder Längenmeßeinrichtung aufweist, die die Zeitdauer bzw. die Länge vom Erfassen der Vorderkante eines Stückes Schnittholz bis zum Auftreten des Informationssignales mißt, wobei die Zeit- oder Längenmeßeinrichtung mit der Speichervorrichtung verbunden ist,

e) die als Interface ausgebildete Anpaßschaltung elektronische Steuerelemente für die Anpassung der optischen Erfassungsvorrichtung oder für die Anpassung der Lichtquelle an unterschiedliche Grundhelligkeiten der Oberfläche des Schnittholzes unter Ausnutzung der maximalen Dynamik der optischen Erfassungsvorrichtung aufweist.

Durch die Abtastung, die sehr schnell durchführbar ist, da sie zeilenweise und ringsherum ausführbar erfolgt, und die Optimierung der angeschlossenen Auswertvorrichtung ist bei gleichzeitig einfachem Aufbau der Vorrichtung eine große Durchlaufgeschwindigkeit des Schnittholzes bei gleichzeitig hoher Prüf- und Störsicherheit erreichbar.

Die optische Erfassungsvorrichtung mit der Lichtquelle ist an einen Tragrahmen angeordnet, der z.B. in sich geschlossen das zu erfassende Schnittholz umgibt, wobei je nach der Erfassungsfläche an dem Tragrahmen und unten und an den Seiten optische Erfassungsvorrichtungen mit Lichtquellen angeordnet sind, wobei auch die Möglichkeit besteht, nur eine einzige Lichtquelle zu verwenden, wobei dann bei dieser Ausführungsform an dem Tragrahmen eine entsprechende Anzahl von Umlenkspiegeln derart angeordnet ist, daß nur bestimmte Flächen des Schnittholzes beleuchtet werden. Dies gilt auch für die optische Erfassungsvorrichtung, die über Umlenkspiegel auf die zu erfassenden Flächen gerichtet ist, so daß neben einer der Anzahl der zu erfassenden Schnittholzflächen entsprechenden Anzahl von optischen Erfassungsvorrichtungen auch nur eine einzige optische Erfassungseinrichtung zum Einsatz gelangen kann, wobei dann die unterschiedlichen Lichtintensitäten der beleuchteten Schnittholzflächen als Unterscheidungsmerkmal dafür herangezogen werden können, welche der Schnittholzflächen während des Augenblicks des Erfassens gerade erfaßt wird, um das erforderliche Informationssignal zu erhalten, wobei auch die Möglichkeit besteht, daß in unterschiedlichen Zeitintervallen die verschiedenen Flächen des Schnittholzes nacheinander erfaßt und die erhaltenen Werte gespeichert werden.

Die Arbeitsweise der Vorrichtung besteht darin, daß das Schnittholz kontinuierlich in seiner Längsrichtung relativ zu einer Lichtquelle und einer optischen Erfassungsvorrichtung bewegt wird, daß die Lichtquelle einen schmalen und sich über die Breite des Schnittholzes erstreckenden Bereich beleuchtet und daß die optische Erfassungsvorrichtung in der Ebene mit der Beleuchtung quer zur Bewegungsrichtung des Schnittholzes mittels einer CCD-Meßzeile zeilenweise abtastet und ansprechend auf Helligkeits- und/oder Farbänderungen des Schnittholzes ein Informationssignal abgibt. Dabei wird die ganze Breite des Schnittholzes z.B. zeilenförmig von der Erfassungsvorrichtung erfaßt. Das Informationssignal wird positionsbezogen und jedem geprüften Stück Schnittholz abgespeichert.

Dadurch, daß ein optisches Verfahren verwendet wird, kann zunächst der Vorteil erreicht werden, daß auch Farbfehler registriert werden können. Es besteht nämlich die Möglichkeit, ansprechend auf Helligkeitsunterschiede oder auf Farbunterschiede oder auf eine Kombination von Helligkeits- und Farbunterschieden ein Informationssignal abzugeben, das die Lage der jeweiligen Fehlstelle kennzeichnen kann.

Durch die genaue Positionierung des Fehlers bzw. der Fehler eines Schnittholzes ist es möglich, das Schnittholz optimal auszuwerten bzw. zu verwerten. Es können nämlich pro Schnittholzstück problemlos die fehlerfreien Längen festgestellt werden, die dann durch eine nachgeschaltete Sägeeinrichtung herausgetrennt werden. So steigt die Ausbeute an fehlerfreiem Holz beträchtlich. Dabei wird eine hohe Prüfgeschwindigkeit erreicht. Beispielsweise kann die Prüfvorrichtung feststehend ausgebildet sein und das Holz an der Prüfvorrichtung vorbeibewegt werden. Holz kann so mit einer Durchlaufgeschwindigkeit von 3 m/s geprüft werden.

Ein besonderer Vorteil besteht darin, daß mit der erfindungsgemäßen Vorrichtung trotz kontinuierlichen Betriebes die Erfassung von Fehlstellen stück- oder lagebezogen erfolgen kann. Es ist möglich, zu jedem gelagerten Holzstück neben der absoluten Länge die fehlerfreie Länge, also die Länge zwischen etwaigen Fehlstellen, abzuspeichern. Wenn nun Schnittholz mit einer bestimmten fehlerfreien Länge verlangt wird, so kann hierfür das geeignetste Stück Schnittholz schnell ermittelt werden.

Praktisch wird es so möglich, auch Schnittholz aus einer nach DIN 68356 vorgegebenen geringeren Güteklasse für höherwertige Zwecke zu verwenden.

Durch die neue Vorrichtung kann auch die bislang durchgeführte Sichtkontrolle des Holzes, die das Auge des Prüfenden stark belastete, völlig entfallen.

Die Abtastung des Holzes erfolgt in Zeilen. Die Zeile erstreckt sich quer zur Längsrichtung des Holzes. Wenn innerhalb einer Zeile eine Fehlstelle erfaßt wird, führt dies zur Abgabe eines Informationssignales. Zusätzlich wird die Position der Fehlstelle, bezogen auf die Längsrichtung des Holzes, abgespeichert. Auf einfache Weise kann aus der zwischen zwei erfaßten Fehlstellen zurückgelegten Wegstrecke die fehlerfreie Länge ermittelt werden. Dabei wird für die optische Abtastung eine CCD-Meßzeile verwendet. Die Ausgangssignale dieser Meßzeile können leicht in einem Rechner aufbereitet und abgespeichert werden. Hierbei kann ein handelsübliches Interface verwendet werden. Ferner ist auch die Helligkeits-Grundeinstellung und die Autofokussierung relativ einfach zu bewerkstelligen. Besonders vorteilhaft ist es, wenn lediglich die Fehlerschwelle, d.h. der Helligkeitsunterschied, ab welchem eine Helligkeitsänderung als Fehler erfaßt werden soll, eingestellt werden muß.

Die Einpegelung auf die Helligkeit des fehlerfreien Holzes kann beispielsweise dadurch erfolgen, daß zunächst in einer Einstell-Betriebsart des Rechners ein fehlerfreier Holzbalken durch die Prüfvorrichtung geschickt wird. Dabei wird dann der durchschnittliche Helligkeitswert erfaßt und als Referenz für die weiteren Messungen verwendet.

Gewünschtenfalls kann auch die Lage der Fehlstelle bezogen auf die Achse der CCD-Zeile od. dgl. mit abgespeichert werden.

Ferner ist es möglich, Schnittholz von vier Seiten zu begutachten, da vier Prüfvorrichtungen links, rechts, oberhalb und unterhalb des zu prüfenden Schnittholzes angeordnet sind. Dadurch, daß das Schnittholz an zwei Kanten anliegen kann, ist lediglich die Verwendung von zwei Autofokussiereinrichtungen an den beiden anderen Prüfeinrichtungen erforderlich.

Die Vorrichtung ermöglicht es, daß Holz nahezu beliebiger Länge und Stärke geprüft werden kann, daß ferner keine Einschränkungen hinsichtlich der verwendeten Holzart bestehen und daß auch die bislang nur durch Inaugenscheinnahme erfaßbaren Farbfehler abgespeichert werden können.

Besonders vorteilhaft ist es ferner, daß die gemessenen Daten auch in einem integrierten System abgespeichert werden können, in welchem neben der Abspeicherung der Fehlstelle, der Qualität, der Abmessungen und des Lagerortes eines jeden Holzstückes auch die für die kaufmännische Abwicklung erforderlichen Maßnahmen durchgeführt werden können.

Vorteilhafte Ausführungsformen sind in den Unteransprüchen angegeben.

Weitere Einzelheiten, Merkmale und Vorteile werden anhand der Beschreibung mehrerer Ausführungsbeispiele der Erfindung näher erläutert. Es zeigt

Fig. 1 eine perspektivische Ansicht einer zur Erfindung führenden Ausführungsform einer Schnittholz-Prüfvorrichtung,

Fig. 2 ein Blockschaltbild einer Ausführungsform einer Meßschaltung für die Schnittholz-Prüfvorrichtung,

Fig. 3 eine schematische Darstellung eines Teiles der Prüfvorrichtung,

Fig. 4 eine Ansicht einer weiteren Ausführungsform der Prüfvorrichtung, und

Fig. 5 Zeitdiagramme zur Darstellung von Meßsignalen, die in der Meßschaltung gemäß Fig. 2 auftreten.

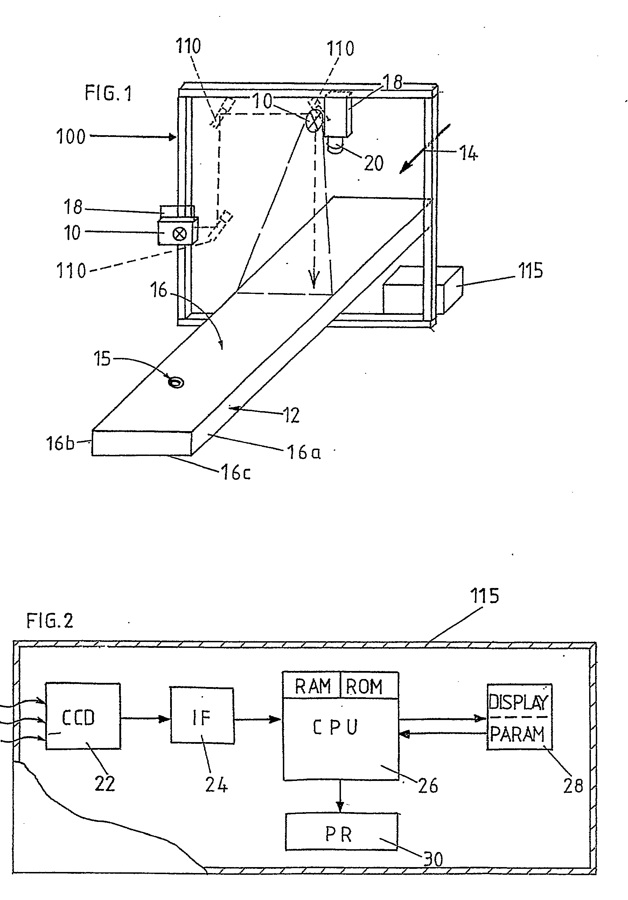

Bei der in Fig. 1 dargestellten Ausführungsform ist eine Lichtquelle 10 vorgesehen, die einen Lichtstrahl nach unten auf ein zu prüfendes Stück Schnittholz 12 wirft. Das Stück Schnittholz 12 ist in seiner Längsrichtung beweglich und wird auf nicht dargestellten Führungseinrichtungen in Richtung des Pfeiles 14 mit einer Geschwindigkeit von z.B. 3 m/s bewegt. Der Lichtstrahl aus der Lichtquelle 10 ist auf die Oberfläche des Holzstückes 12 fokussiert, wobei der Lichtstrahl quer zur Längsrichtung des Holzstücks 12 kegelförmig, zeilenförmig oder gescannt aufgeweitet ist, während er in Längsrichtung des Holzstückes 12 stärker fokussiert ist. Dadurch wird eine relativ hohe Lichtintensität erzielt.

Die Lichtquelle 10 weist einen derartigen Abstand von der Oberfläche 16 des Holzstücks 12 und damit gegebenenfalls auch von der Fehlstelle 15 auf, daß eine gleichmäßige Ausleuchtung erzielt wird. Unmittelbar benachbart zu der Lichtquelle 10 ist eine optische Erfassungsvorrichtung 18 angeordnet. Die optische Erfassungsvorrichtung 18 weist ein Objektiv 20 auf, das ebenfalls auf die Oberfläche 16 fokussiert ist. Die Anordnung von Lichtquelle 10 und Objektiv 20 zueinander ist so gewählt, daß keine Meßfehler, insbesondere keine Parallaxenfehler, auftreten. Dann kann auch bei einer relativ großen Transportgeschwindigkeit des Holzes eine Auflösung in der Größenordnung von 1 mm erzielt werden.

Während der Bewegung des Holzes wird die Oberfläche 16 von der optischen Erfassungsvorrichtung 18 zeilenweise abgetastet. Pro Zeile stehen dabei 0,3 ms zur Verfügung. Für die Umwandlung der optischen in die elektrischen Signale kommen daher schnelle Halbleiter-Zeilenkameras in Frage. Bei reduzierten Geschwindigkeits- bzw. Auflösungs-Anforderungen können aber auch preiswertere optoelektrische Wandler Verwendung finden. Auch andere Abtastarten, wie z.B. eine punktförmige Abtastung oder eine Array-Abtastung, können zur Anwendung gelangen.

Es besteht die Möglichkeit, ein optisches und/oder ein elektrisches Filter für die optische Erfassungsvorrichtung 18 vorzusehen. Damit können Störeinflüsse und Verfälschungen des Meßergebnisses ausgeblendet werden.

Wie Fig. 1 weiter zeigt, ist im Vorschubbereich des zu erfassenden Schnittholzes 12 ein Tragrahmen 100 angeordnet, der Bestandteil der Schnittholz-Prüfvorrichtung ist und die Erfassungsvorrichtung 18 und die Lichtquelle 10 aufnimmt. Dieser Tragrahmen 100 kann ein U-förmiges Profil mit seitlichen Längsholmen, die dann gleichzeitig die Standfüße des Tragrahmens bilden, und einem oberen Querholm aufweisen, der dann die Erfassungsvorrichtung 18 mit der Lichtquelle 10 trägt, wenn die Oberfläche 16 des Schnittholzes 12 erfaßt werden soll. Für die erfindungsgemäße Erfassung der Seitenflächen 16a, 16b sind an den senkrechten Längsholmen des Tragrahmens Erfassungsvorrichtungen und Lichtquellen vorzusehen. Um auch die untere Fläche 16c des Schnittholzes 12 zu erfassen, wird ein in sich geschlossener Tragrahmen 100, wie in Fig. 1 dargestellt, zur Anwendung gelangen, der auch an seinem unteren Querholm eine Erfassungsvorrichtung und eine Lichtquelle trägt.

Um mehr als eine Fläche des Schnittholzes 12 zu erfassen, braucht nicht jede Schnittholzfläche mittels einer Lichtquelle beleuchtet zu werden. Es ist eine Lichtquelle ausreichend, in deren Strahlengang an dem Tragrahmen 100 eine entsprechende Anzahl von Umlenkspiegeln 110 derart angeordnet ist, daß auch mehrere Flächen des Schnittholzes beleuchtet werden können. Bei dem in Fig. 1 gezeigten Beispiel wird mittels der an dem Tragrahmen 100 angebrachten Umlenkspiegel die Oberfläche 16 des Schnittholzes beleuchtet. Werden über eine Lichtquelle 10 mehrere Flächen des Schnittholzes beleuchtet, dann ist jeder beleuchteten Schnittholzfläche eine Erfassungsvorrichtung 18 zugeordnet. Es besteht aber auch die Möglichkeit, über ein Umlenkspiegelsystem mit nur einer einzigen Erfassungsvorrichtung 18 mehrere Schnittholzflächen zu erfassen. Über unterschiedliche Lichtintensitäten, Länge des zurückgelegten Lichtstrahles oder Beobachtungsstrahles können die einzelnen Schnittholzflächen festgelegt und einzeln erfaßt und die erfaßten Werte so gespeichert werden, daß sie den einzelnen Schnittholzflächen später zugeordnet werden können, wobei auch die Möglichkeit besteht, in schneller Zeitfolge und wiederkehrenden Reihenfolgen die einzelnen verschiedenen Schnittholzflächen zu erfassen.

Als Lichtquelle 10 können alle geeigneten Lichtquellen verwendet werden, insbesondere monochromatisches Licht, Laser-Licht od. dgl.

Der die Erfassungsvorrichtung 18 und die Lichtquelle 10 aufnehmende Tragrahmen 100 nimmt darüber hinaus auch alle weiteren Teile der Schnittholz-Prüfvorrichtung auf. Die Meßschaltung ist dann in einem Gehäuse 115 angeordnet, das mit dem Tragrahmen 100 verbunden ist.

In Fig. 2 ist die einen CCD-Scanner 22 als Wandler enthaltende Meßschaltung dargestellt. Der CCD-Scanner 22 ist über ein geeignetes Interface 24 mit einem Mikrocomputer 26 verbunden, der eine zentrale Verarbeitungseinheit CPU, einen Schreib/Lese-Speicher RAM und einen Programmspeicher ROM aufweist. Das Interface 24 dient als Anpaßschaltung zwischen dem CCD-Scanner 22 und dem Mikrocomputer 26. Der Mikrocomputer 26 weist ferner eine Bedieneinheit 28 auf, die die Anzeige der gemessenen Fehlstellen erlaubt und die Eingabe von Parametern für die Steuerung der Fehlererfassung ermöglicht. Ferner ist ein Drucker 30 vorgesehen, der dazu geeignet ist, ein Protokoll der geprüften Hölzer zu liefern, das zugleich zur Erfassung des Eingangs in das Lager dienen kann.

Es findet ein CCD-Scanner 22 mit 500 bis 2000 Pixel Verwendung. Mit einem derartigen Scanner ist es möglich, auch größere Holzoberflächen abzutasten. Die Abtastung selbst erfolgt in der bekannten Weise, so daß das Eingangssignal für das Interface 24 in serieller Form vorliegt. In dem Interface 24 wird eine A/D-Wandlung vorgenommen. Das jeder Abtastperiode zugehörige Meßsignal wird in digitalisierter Form dem Mikrocomputer 26 zugeführt. Der Mikrocomputer 26 prüft, ob innerhalb der gemessenen CCD-Zeile ein Meßwert die Fehlerwertschwelle überschreitet. Besonders vorteilhaft ist es hierbei, wenn eine bei lediglich einem einzigen Pixel auftretende Fehlerwertanzeige ausgeblendet wird, da es sich dann höchstwahrscheinlich lediglich um eine Reflexion an einer Holzfaser handelt.

Ferner ist eine Auflösung von 8 bit für den A/D-Wandler ausreichend, so daß auf preiswerte Technik zurückgegriffen werden kann. Der Mikrocomputer kann auch als Single-Chip-Baustein mit integrierter Ansteuerung der Peripheriegeräte ausgebildet sein kann, was eine weitere Kostenreduzierung ermöglicht. Zur Sicherstellung eines störungsfreien Betriebes auch unter rauhen Umweltbedingungen kann die Elektronik in – zudem stromsparender – CMOS-Technik ausgeführt sein.

Der in dem Mikrocomputer 26 verwendete Halbleiterspeicher RAM kann sowohl zur Zwischenspeicherung der erfaßten Fehlerstellen unter Berücksichtigung ihrer Größe und Lage als auch als reiner CPU-bezogener Arbeitsspeicher ausgebildet sein. Die Meßschaltung kann auch als Front-End-Prozessor in einem Netzwerk, besonders vorteilhaft mit Sternstruktur, eingesetzt werden. Im Stand-Alone-Betrieb kommt jedoch auch der Einsatz eines zusätzlichen Hintergrundspeichers in Betracht.

Die in Fig. 2 dargestellte Ausführungsform der Meßschaltung erlaubt die vollautomatische Prüfung von Schnittholz. Die Schaltung wird auf einen Wert eingestellt, der der Grundhelligkeit des verwendeten Holzes entspricht. Hierzu wird zunächst in der Einstell-Betriebsart des Mikrocomputers 26 ein fehlerfreies oder nahezu fehlerfreies Stück Holz der zu überprüfenden Art gemessen. Dessen Helligkeits-Durchschnittswert wird von dem Mikrocomputer 26 berechnet und für die Grundeinstellung übernommen. Dann wird in die Meß-Betriebsart umgeschaltet und eine Einstellung der gewünschten Fehlerschwelle vorgenommen. Die Fehlerschwelle ist in der Regel abhängig von der gewünschten Qualitätstoleranz und damit unter anderem von der verwendeten Holzart. Dann kann die ganze Charge mit hoher Geschwindigkeit geprüft und die Fehlerstellen abgespeichert werden.

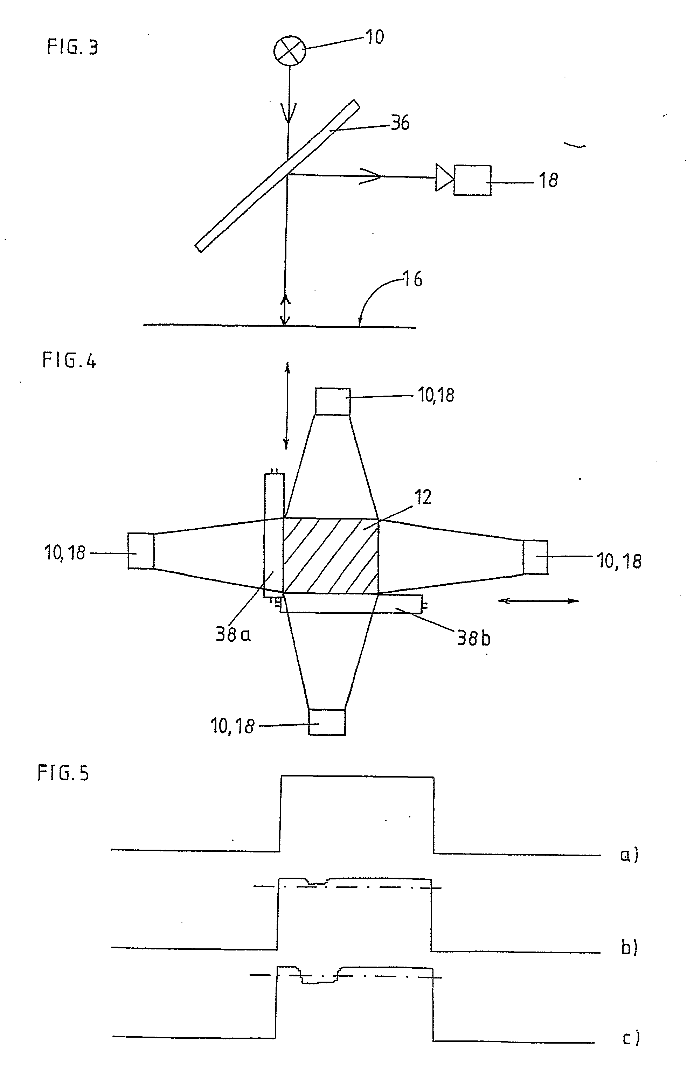

In Fig. 3 ist eine besonders vorteilhafte Ausbildung der optischen Erfassungsvorrichtung 18 in Verbindung mit der Lichtquelle 10 dargestellt. Es wird ein halbdurchlässiger Spiegel 36 verwendet, der im Lichtstrahl von der Lichtquelle 10 zur Oberfläche 16 schräg angeordnet ist. Nach Durchtreten des halbdurchlässigen Spiegels 36 gelangt der von der Lichtquelle 10 kommende Lichtstrahl auf die Oberfläche 16 und wird dort reflektiert. Er trifft auf die Unterseite des halbdurchlässigen Spiegels 36 und wird dort reflektiert und der optischen Erfassungsvorrichtung 18 zugeleitet. Diese Ausführung erlaubt eine besonders genaue Messung der Lage der Fehler.

In Fig. 4 ist eine weitere Ausführungsform einer Prüfeinrichtung dargestellt. Das zu prüfende Stück Schnittholz 12 ist ein Vierkant, dessen Flächen alle untersucht werden müssen. Hierzu sind vier Lichtquellen mit vier optischen Erfassungsvorrichtungen je über einer der Schnittflächen angeordnet. Lediglich zwei der Erfassungsvorrichtungen sind mit einer Autofokussiermechanik versehen, da der Abstand zur Holzoberfläche bei den beiden anderen Erfassungsvorrichtungen aufgrund der verwendeten Führungsrollen 38a und 38b stets gleich ist.

In Fig. 5 ist bei a) das Ausgangssignal einer optischen Erfassungsvorrichtung beim einzeiligen Abtasten eines fehlerfreien Holzstücks dargestellt. Bei b) ist eine Fehlstelle erreicht, die sich durch eine geringere Amplitude in einem Teilbereich des Meßsignals bemerkbar macht. Hier unterschreitet die Amplitude jedoch noch nicht den Fehler-Schwellenwert. Bei c) ist das Meßsignal während des Abtastens einer Meßzelle dargestellt, welche die Fehlstelle voll erfaßt. Es wird ein Informationssignal abgegeben und die Position der Fehlstelle abgespeichert. Die längenbezogene Abspeicherung erfolgt dadurch, daß von dem Mikrocomputer 26 die Zeit zwischen der Vorderkante des gerade durchlaufenden Stücks Schnittholz bis zum Auftreten des Informationssignales gemessen wird. Diese Zeit ist wegen der konstanten Vorschubgeschwindigkeit längenproportional und entspricht damit der Länge des fehlerfreien Teils des Holzstücks.

## Patentansprüche

1. Schnittholz-Prüfvorrichtung für die laufende Erfassung von gesägtem Schnittholz (12), mit mindestens einer Lichtquelle (10), die das Schnittholz (12) in einer Ebene quer zu seiner Längsrichtung (14) mit im wesentlichen gleicher Intensität beleuchtet, mit mindestens einer optischen Erfassungsvorrichtung (18), die ansprechend auf Helligkeits- und/oder Farbänderungen in der von der Erfassungsvorrichtung (18) erfaßten beleuchteten Ebene des Schnittholzes (12) während einer Relativbewegung zwischen Schnittholz (12) und optischer Erfassungsvorrichtung (18) ein Informationssignal abgibt, und mit einer mit der Erfassungsvorrichtung (18) verbundenen Rechenvorrichtung (26), mit der positionsbezogene Informationssignale zu jedem geprüften Stück Schnittholz sequentiell abspeicherbar sind, wobei eine Anpaßschaltung (24) vorgesehen ist, mit der eine Fehlerschwelle für die Erzeugung des Informationssignals einstellbar ist, gekennzeichnet durch die Kombination folgender Merkmale:

a) die Schnittholz-Prüfvorrichtung umfaßt einen Tragrahmen (100), an dem eine obere optische Erfassungseinrichtung (18) und zwei seitliche optische Erfassungsvorrichtungen (18) mit je einer Lichtquelle (10) und eine untere optische Erfassungsvorrichtung (18) mit einer weiteren Lichtquelle (10) angeordnet sind, oder umfaßt einen Tragrahmen (100), der eine Lichtquelle (10), eine optische Erfassungsvorrichtung (18) und eine Anzahl von Umlenkspiegeln (110) trägt, die derart an dem Tragrahmen (100) angeordnet sind, daß die Lichtquelle (10) das Schnittholz (12) mit im wesentlichen gleicher Intensität beleuchtet, wobei die optische Erfassungsvorrichtung (18) über das Umlenkspiegelsystem die beleuchteten Ebenen des Schnittholzes erfaßt,

b) die jeweilige Lichtquelle (10) beleuchtet das Schnittholz (12) in der Ebene quer zu seiner Längsrichtung (14) kegelförmig, wobei ein schmaler und sich über die jeweilige Breite des Schnittholzes erstreckender Bereich beleuchtet wird,

c) in dieser Ebene ist die mittels einer CCD-Meßzeile (22) zeilenweise abtastende optische Erfassungsvorrichtung (18) vorgesehen,

d) die Rechenvorrichtung (26) weist eine Zeit- oder Längenmeßeinrichtung auf, die die Zeitdauer bzw. die Länge vom Erfassen der Vorderkante eines Stückes Schnittholz (12) bis zum Auftreten des Informationssignales mißt, wobei die Zeit- oder Längenmeßeinrichtung mit der Speichervorrichtung verbunden ist,

e) die als Interface ausgebildete Anpaßschaltung (24) weist elektronische Steuerelemente für die Anpassung der optischen Erfassungsvorrichtung (18) oder für die Anpassung der Lichtquelle an unterschiedliche Grundhelligkeiten der Oberfläche des Schnittholzes (12) unter Ausnutzung der maximalen Dynamik der optischen Erfassungsvorrichtung auf.

2. Schnittholz-Prüfvorrichtung nach Anspruch 1, dadurch gekennzeichnet, daß die optische Erfassungsvorrichtung (18) ein Filter für die Erhöhung der Empfindlichkeit auf bestimmte Holzfehler aufweist.

3. Schnittholz-Prüfvorrichtung nach Anspruch 1 oder 2, dadurch gekennzeichnet, daß die Speichereinrichtung als integriertes System ausgebildet ist, in dem die Abspeicherung der Fehlstelle, der Qualität, der Abmessungen und des Lagerortes erfolgt.

## Revendications

1. Dispositif de contrôle pour bois de sciage our la détection continue de bois de sciage (12), avec au moins une source de lumière (10) qui éclaire le bois de sciage (12) dans un plan transversal par rapport à son sens longitudinal (14) avec une intensité substantiellement égale, avec au moins un dispositif de détection optique (18) qui donne un signal d'information en réagissant aux changements de luminosité et/ou de couleur du plan éclairé du bois de sciage (12) détecté par le dispositif de détection (18) pendant un mouvement relatif entre le bois de sciage (12) et le dispositif de détection optique (18), et avec un dispositif de traitement des données (26) relié au dispositif de détection (18) avec lequel des signaux d'information aux positionnements référencés peuvent être mis en mémoire de manière séquentielle pour chaque pièce de bois de sciage contrôlée, un circuit d'adaptation (24) étant prévu, avec lequel un seuil de défauts est réglable pour la génération des signaux d'information, caractérisé par la combinaison des caractéristiques suivantes:

a) le dispositif de contrôle pour bois de sciage comprend un cadre porteur (100) sur lequel sont disposés un dispositif supérieur de détection optique (18) et deux dispositifs latéraux de détection optique (18) avec chacun une source lumineuse (10) et un dispositif inférieur de détection optique (18) avec une autre source de lumière (10) ou comprend un cadre porteur (100) qui porte une source de lumière (10), un dispositif de détection optique (18) et un certain nombre de miroirs de déviation (110) qui sont disposés sur le cadre porteur (100) de manière telle que la source de lumière (10) éclaire le bois de sciage (12) avec une intensité substantiellement égale, le dispositif de détection optique (18) détectant les plans éclairés du bois de sciage par l'intermédiaire du système de miroirs de déviation:

b) la source lumineuse respective (10) éclaire le bois de sciage (12) dans le plan transversal par rapport à son sens longitudinal (14) de manière conique en éclairant une zone étroite s'étendant sur la largeur respective du bois de sciage;

c) dans ce plan est prévu le dispositif de détection optique (18) explorant ligne par ligne au moyen d'une ligne de mesure à CCD (22);

d) le dispositif de traitement des données (26) présente un dispositif de mesure du temps et de la longueur qui mesure la durée et/ou la longueur depuis la détection de l'arête antérieure d'une pièce de bois de sciage (12) jusqu'à l'apparition du signal d'information, le dispositif de mesure du temps et de la longueur étant relié au dispositif de mémorisation;

e) le circuit d'adaptation (24), conçu comme étant une interface, présente des éléments électroniques de commande pour l'adaptation du dispositif de détection optique (18) ou pour l'adaptation de la source lumineuse aux différentes luminosités de base de la surface du bois de sciage (12) en utilisant la dynamique maximale du dispositif de détection optique.

2. Dispositif de contrôle pour bois de sciage selon la revendication 1, caractérisé en ce que le dispositif de détection optique (18) présente un filtre pour augmenter la sensibilité à certains défauts du bois.

. 3. Dispositif de contrôle pour bois de sciage selon la revendication 1 ou 2, caractérisé en ce que le dispositif de mémorisation est configuré comme un système intégré dans lequel a lieu la mémorisation du défaut, de la qualité, des dimensions et de sa situation.

## Claims

1. A sawn timber testing system for the continuous detection of sawn timber (12), with at least one light source (10) for illuminating the sawn timber (12) in a plane transversely to its longitudinal direction (14) with substantially uniform intensity, with at least one optical detection device (18) which, in response to changes in brightness and/or in colour of the illuminated plane of the sawn timber (12) determined by the detection device (18) during a relative moment between the sawn timber (12) and the optical detection device (18), emits an information signal, and with a computing device (26) coupled to the detection device, with the aid of which position-related information signals regarding each tested piece of sawn timber can be sequentially stored, in which an adapter circuit (24) is provided with the aid of which an error threshold for the generation of the information signal can be adjusted, characterized by the combination of the following features:

a) The sawn timber testing system comprises a supporting frame (100), on which an upper optical detection device (18) and two lateral optical detection devices (18) are mounted with one light source (10) each, and a lower optical detection device (18) with a further light source (10), or comprises a supporting frame (100) bearing an optical detection device (18) and a pluraliy of reflecting mirrors (110) which are disposed on the supporting frame (100) in such a way that the light source (10) illuminates the sawn timber with substantially uniform intensity, while the optical detection device (18) covers the illuminated planes of the sawn timber by means of the reflecting mirror system;

b) The light source (10) in question illuminates the sawn timber (12) within the plane transversely to its longitudinal direction (14) in a conical manner, a narrow area extending across the respective width of the sawn timber;

c) The optical detection device (18) line-wise scanning with the aid of a CCD measuring line (22) is provided within its plane;

d) The computing device (26) possesses a period or length of time measuring means which measures the length of time or the period from the detection to the front edge of a piece of sawn timber (12) until the occurrence of the information signal, the period or length of time measuring means being coupled to the storage device;

e) The adapter circuit (24) constructed in the form of an interface is provided with an electronic control components for the adaptation of the optical detection device (18) or for the adaptation of the light source to different basic brightnesses of the surface (12) of the sawn timber while utilizing the maximum dynamics of the optical detection device.

2. A sawn timber testing system according to claim 1, characterized in that the optical detection device (18) possesses a filter for increasing the sensitivity to certain timber flaws.

3. A sawn timber testing system according to claim 1 or 2, characterized in that the storage device is constructed in the form of of an integrated system in which the storage of the location of the flaw, the qualitiy, the dimensions and of the timber yard location is effected.

FIG.1

FIG.2

FIG. 3

10

36

18

16

FIG. 4

10,18

12

10,18

10,18

38a

38b

10,18

FIG. 5

a)

b)

c)